# EUROPEAN PATENT APPLICATION

(11) **EP 3 901 285 A1**
(43) Date of publication of application: **27.10.2021**
(21) Application number: 20305400.2
(22) Date of filing: 23.04.2020
(51) Int. Cl.: C12Q 1/6806

(54) **PROCESS FOR EXTRACTING DOUBLE-STRANDED DNA**

(71) Applicant: Centre National de la Recherche Scientifique, 75016 Paris (FR); Université Côte d'Azur, 06100 Nice Cedex 2 (FR); Université Savoie Mont Blanc, 73000 Chambéry (FR); Université Grenoble Alpes, 38400 Saint-Martin-d'Hères (FR); Institut Polytechnique de Grenoble, 38000 Grenoble (FR)
(72) Inventor: SZYMANSKI - MARTINET, Nadine, 06300 NICE (FR); PAPAICONOMOU, Nicolas, 06300 NICE (FR); BARNOIN, Guillaume, 06100 NICE (FR); MICHEL, Benoît, 06510 GATTIERES (FR); BURGER, Alain, 06000 NICE (FR)
(74) Representative: Cabinet Becker et Associés

(57) **Abstract**

The present invention relates to a process for extracting double-stranded DNA, using ionic liquids of formula (I). It also relates to kits comprising such ionic liquids to implement such a process.

## Description

### TECHNICAL FIELD

The present invention relates to a process for extracting double-stranded DNA from biological samples, using ionic liquids. It also relates to kits comprising such ionic liquids to implement such a process.

### TECHNICAL BACKGROUND

Tremendous breakthroughs in the genetic field have been achieved over the last decades, and many procedures such as DNA sequence analysis, genetic recombination, or clinical hybridization are implemented in research laboratories, nowadays. A simple and efficient access to high purity DNA is crucial for such procedures to be successful.

Conventional methods for separating and purifying DNA from a biological sample are mostly column-based or are using functionalized magnetic particles. Such methods are however expensive and their average DNA extraction yields do not exceed 50 %. Also, manual methods imply a high number of handling steps and use of toxic solvents.

Due to their unusual properties, ionic liquids have been the topic of many research projects. Ionic liquids have been applied to a wide diversity of fields, such as polymerization, catalysis or electrochemistry, and their great potential in biotechnological processes is now recognized. More specifically, such particular compounds have been used for stabilizing, extracting and/or purifying biological molecules, such as DNA.

Imidazolium salts have been used for extracting DNA. Nacham et al. (Anal. Chem. 2016, 88, 7813) describes a solid phase DNA extraction process coupled with quantitative PCR, using an absorbing solid phase comprising imidazolium-type ionic liquid moieties. However, this process requires the use of a sophisticated solid phase material, and low extraction yields are obtained with such a process. Wang et al. (Anal. Chem. 2007, 79, 620-625) describes a process for extracting double-stranded DNA, using 1-butyl-3-methylimidazolium hexafluorophosphate. However, low extraction yields are obtained with such a process.

Pyridinium salts have also been used for stabilizing and/or extracting DNA. WO 2015/120447 describes a process for extracting genomic or plasmid DNA. Such a process comprises contacting a biological sample with a phase-separation solution containing a detergent, capturing DNA with a mineral matrix, such as a silica matrix, treating and washing the captured DNA. The detergent can, among others, be cetylpyridinium chloride or dodecylpyridinium chloride. However, such detergents are not highly selective and tend to form aggregate with proteins, which reduces the efficacy of the process for extracting DNA.

US 2018/334703 describes a stabilizing agent for storing and preserving a nucleic acid, such as DNA or RNA. The stabilizing agent is a pyridinium salt, and can in particular be octylpyridinium bromide, butylpyridinium bromide or phenacylpyridinium bromide. In order to extract the nucleic acid stored in the stabilizing agent, a use of a commercially available kit comprising silica membranes, which is quite expensive.

Thus, there remains a need for providing a simple, cost-effective, and selective process for extracting double-stranded DNA with good yields, while preserving the native structure of DNA.

### SUMMARY OF THE INVENTION

In this context, the inventors have developed an efficient process for extracting double-stranded DNA using an ionic liquid-type compound of formula (I). The compounds of formula (I) are pyridinium salts that allow obtaining double-stranded DNA in large amounts, while preserving its native structure. The process of the invention comprises mere steps, including DNA precipitation and condensation steps, using readily available chemical reagents. The process of the invention is therefore easy to implement and safe for the user.
Contrary to the processes known in the art, the process of the invention is highly selective, allowing thereby to obtain pure samples of double-stranded DNA, which are thus substantially deprived of other biological molecules such as RNA or single-stranded DNA.

Hence, the present invention relates to a process for extracting double-stranded DNA, comprising the following steps:
(a) reacting a biological sample comprising double-stranded DNA with a compound of formula (I): 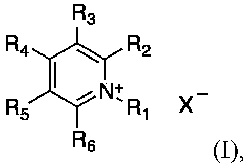 in which:
   - Ri represents a C₅-C₁₁ alkyl group,
   - R₂, R₃, R₄, Rs and R₆ represent each independently:
      - a hydrogen atom, a halogen atom, -OH, -CN, -NO₂, -SH, -NR'R" with R' and R" being independently a hydrogen atom or a (C₁-C₆)alkyl, or
      - a radical selected from the group consisting of a (C₁-C₆)alkyl, a (C₂-C₆)alkenyl, (C₂-C₆)alkynyl, a (C₁-C₆)alkoxy, a (C₃-C₁₂)cycloalkyl, a (C₃-C₁₂)heterocycloalkyl, an aryl, and a heteroaryl, said radical being optionally substituted by at least one -OH, (Ci-C₆)alkoxy, (C₁-C₆)alkanoyl, or -NR'R" with R' and R" being independently a hydrogen atom or a (C₁-C₆)alkyl, and
   - X⁻ represents an anion; and
(b) recovering double-stranded DNA.

In a particular embodiment, Ri represents a C₇-C₁₀ alkyl group, preferably an octyl group.
In another particular embodiment, R₂, R₃, R₄, R₅ and R₆ represent each independently a hydrogen atom, or a (C₁-C₆)alkyl optionally substituted by -OH, -NH₂, (C₁-C₆)alkoxy, or (Ci-C₆)alkanoyl.
In a further particular embodiment, R₂, R₃, R₄, R₅ and R₆ represent each independently a hydrogen atom or a methyl.
In another particular embodiment, X⁻ is a halide, a dicyanamide, a nitrate, a cyanate, a thiocyanate, a (C₁-C₆)alkanoate, a (C₁-C₆)alkylsulfonate, a benzenesulfonate or toluenesulfonate. Preferably, X⁻ represents a halide, more preferably a bromide or a chloride, and even more preferably a bromide.
Preferably, a compound of formula (I) is a compound of formula (Ia) or a compound of formula (Ib):

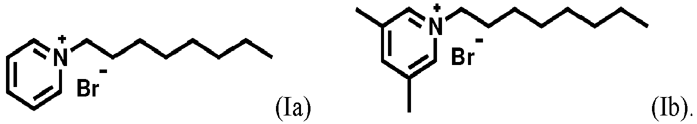

In a particular embodiment, the process of the invention comprises the following steps:
(a) reacting a biological sample comprising double-stranded DNA with a compound of formula (I) as defined herein, to form an aggregate;
(b1) purifying the aggregate obtained in step (a); and
(b2) contacting the aggregate obtained in step (b1) with a buffer solution.
In a particular embodiment, said process further comprises:
(b3) contacting the mixture obtained in step (b2) with at least one alcohol solution.

In a particular embodiment, said buffer solution is a phosphate-buffered saline solution.
In a particular embodiment, the alcohol solution in step (b3) comprises ethanol.
In a particular embodiment, the alcohol concentration of said at least one alcohol solution is greater than or equal to 70 % (v/v).

In another particular embodiment, the double-stranded DNA concentration is between 1 and 20 ng/mL, preferably between 5 and 10 ng/mL.
In another particular embodiment, the double-stranded DNA has more than 50 base pairs, preferably more than 100 base pairs, more preferably more than 300 base pairs, even more preferably more than 1000 base pairs.

The present invention also relates to a use of a compound of formula (I) as defined herein for extracting double-stranded DNA.

Another object of the present invention is a kit for extracting double-stranded DNA comprising:
- a composition comprising a compound of formula (I) as defined herein;
- a buffer solution, preferably a phosphate-buffered saline solution;
- at least one alcohol solution, preferably comprising ethanol; and optionally
- an instruction guide.

### FIGURES

**Figure 1****:** UV spectra of 10 µg Ct-DNA (starting DNA), and 10 µg Ct-DNA extracted by the process of the invention. Absorbance (A) was measured for 300 µM aqueous solution of Compound (Ia) and for the first, second, third and fourth supernatants obtained during the process.
**Figure 2****:** Photographs of samples obtained at different stages of the process of the invention
   **Figure 2A****:** 900 µL samples of mouse serum or plasma were mixed with 100 µL of an aqueous solution of 1 M Compound (Ia) (after the first centrifugation);
   **Figure 2B****:** 900 µL of lysed bacterial plasmids mixed with 100 µL 1M Compound (Ia) aqueous solution (right tube) or with 100 µL 1M Cetyl pyridinium aqueous solution (left tube), after the first centrifugation;
   **Figure 2C****:** MCF7 cells digest after the last centrifugation.
**Figure 3****:** Circular dichroism spectra of 2 µg of Ct-DNA (starting DNA), and 2 µg Ct-DNA duplicate extracted by the process of the invention using Compound (Ia).
**Figure 4****:** Agarose gel electrophoresis of Ct-DNA (B) and duplicate (C) extracted by the process of the invention using Compound (Ia) (A = Control).
**Figure 5****:** Quality evaluation via qPCR of DNA extracted by the process of the invention (left part: DNA from mice kidneys obtained by the process of the invention; right part: phenol/chloroform/isoamyl alcohol purified duplicate DNA).
**Figure 6****:** Scheme illustrating a process for extracting double-stranded DNA according to the invention.

### DETAILED DESCRIPTION OF THE INVENTION

### Compounds of formula (I)

The words mentioned herein with a prefix such as C₁-C₁₂ can also be used with lower numbers of carbon atoms such as C₁-C₂, C₁-C₉, or C₂-C₅. If, for example, the wording C₁-C₁₂ is used for an aliphatic chain, then said aliphatic chain may comprise from 1 to 12 carbon atoms, especially 1, 2, 3, 4, 5, 6, 7, 8, 9, 10, 11, or 12 carbon atoms.
As used herein, "alkyl" refers to a saturated, linear or branched, acyclic hydrocarbon group. Examples of C₁-C₆ alkyl include, but are not limited to, methyl, ethyl, propyl, isopropyl, butyl, tert-butyl, pentyl, or hexyl. Examples of C₅-C₁₁ alkyl include, but are not limited to, pentyl, hexyl, heptyl, octyl, nonyl, decyl, or undecyl. Examples of C₇-C₁₀ alkyl include, but are not limited to, heptyl, octyl, nonyl, or decyl.
As used herein, "alkenyl" refers to an unsaturated, linear or branched aliphatic group comprising at least one carbon-carbon double bond. Examples of "(C₂-C₆)alkenyl" include, but are not limited to, ethenyl, propenyl, isopropenyl, butenyl, isobutenyl, pentenyl, or hexenyl.

As used herein, "alkynyl" refers to an unsaturated, linear or branched aliphatic group comprising at least one carbon-carbon triple bond. Examples of "(C₂-C₆)alkynyl" include, but are not limited to, ethynyl, propynyl, isopropynyl, butynyl, isobutynyl, pentynyl, or hexynyl. As used herein, "cycloalkyl" corresponds to a saturated or unsaturated mono-, bi- or tri-cyclic alkyl group comprising between 3 and 12 carbon atoms. It also includes fused, bridged, or spiro-connected cycloalkyl groups. The term "cycloalkyl" includes for instance cyclopropyl, cyclobutyl, cyclopentyl, and cyclohexyl. The term "cycloalkyl" may also refer to a 5-10 membered bridged carbocyclyl such as bicyclo[2,2,1]heptanyl, bicyclo[2,2,2]octanyl, or adamantyl, preferably bicyclo[2,2,1]heptanyl. Preferably, the "cycloalkyl" is a cyclopropyl, cyclobutyl, cyclopentyl or a cyclohexyl.
As used herein, "heterocycloalkyl" corresponds to a saturated or unsaturated cycloalkyl group as above defined further comprising at least one heteroatom such as nitrogen, oxygen, or sulphur atom. It also includes fused, bridged, or spiro-connected heterocycloalkyl groups. Representative heterocycloalkyl groups include, but are not limited to 3-dioxolane, benzo [1,3] dioxolyl, azetidinyl, oxetanyl, pyrazolinyl, pyranyl, thiomorpholinyl, pyrazolidinyl, piperidyl, piperazinyl, 1,4-dioxanyl, imidazolinyl, pyrrolinyl, pyrrolidinyl, piperidinyl, imidazolidinyl, morpholinyl, 1,4-dithianyl, pyrrolidinyl, oxozolinyl, oxazolidinyl, isoxazolinyl, isoxazolidinyl, thiazolinyl, thiazolidinyl, isothiazolinyl, isothiazolidinyl, dihydropyranyl, tetrahydropyranyl, tetrahydrofuranyl, and tetrahydrothiophenyl. The term "heterocycloalkyl" may also refer to a 5-10 membered bridged heterocyclyl such as 7-oxabicyclo[2,2,1]heptanyl. In a particular embodiment, it may also refer to spiro-connected heterocycloalkyl groups or spiroheterocycloalkyl groups such as for instance oxetanyl spiro-connected with azetidinyl or piperidinyl.
As used herein, "aryl" corresponds to a mono- or bi-cyclic aromatic hydrocarbon having from 6 to 14 carbon atoms. For instance, the term "aryl" includes phenyl, biphenyl, or naphthyl. In a preferred embodiment, the aryl is a phenyl.
As used herein, "heteroaryl" as used herein corresponds to an aromatic, mono- or poly-cyclic group comprising between 5 and 14 atoms and comprising at least one heteroatom such as nitrogen, oxygen or sulphur atom. Examples of such mono- and poly-cyclic heteroaryl group may be: pyridinyl, thiazolyl, thiophenyl, furanyl, pyrrolyl, pyrazolyl, imidazolyl, triazolyl, tetrazolyl, benzofuranyl, thianaphthalenyl, indolyl, indolinyl, quinolinyl, isoquinolinyl, benzimidazolyl, tetrahydroquinolinyl, tetrahydroisoquinolinyl, triazinyl, thianthrenyl, isobenzofuranyl, chromenyl, xanthenyl, phenoxanthinyl, isothiazolyl, isoxazolyl, pyrazinyl, pyridazinyl, indolizinyl, isoindolyl, indazolyl, purinyl, quinolizinyl, phtalazinyl, naphthyridinyl, quinoxalinyl, quinazolinyl, cinnolinyl, pteridinyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, indolinyl, isoindolinyl, oxazolidinyl, benzotriazolyl, benzoisoxazolyl, oxindolyl, benzoxazolinyl, benzothienyl, benzothiazolyl, isatinyl, dihydropyridyl, pyrimidinyl, s-triazinyl, oxazolyl, or thiofuranyl.
As used herein, "alkoxy" or "alkyloxy" corresponds to an alkyl group as defined above attached to the rest of the molecule through a -O- bond. Examples of (C₁-C₆)alkoxy are methoxy, ethoxy, propyloxy, butyloxy, isopropyloxy, tertbutoxy, pentyloxy, hexyloxy. A preferred (Ci-C₆)alkoxy is methoxy.
As used herein, "alkanoyl" corresponds to an alkyl group as defined above attached to the rest of the molecule through a -C(O)- bond. Examples of (C₁-C₆)alkanoyl are ethanoyl (also named "acetyl"), propanoyl, butanoyl, pentanoyl, hexanoyl. A preferred (C₁-C₆)alkanoyl is acetyl. As used herein, "halogen" corresponds to a fluorine, chlorine, bromine, or iodine atom, preferably fluorine, chlorine or bromine.

As used herein, an "alkanoate" anion refers to an anion of formula (alkyl)-CO₂⁻, where alkyl is as defined above. Examples of (C₁-C₆)alkanoate include, but are not limited to, ethanoate (also named "acetate"), propanoate, butanoate, pentanoate, or hexanoate. A preferred (Ci-C₆)alkanoate is acetate.
As used herein, an "alkylsulfonate" anion refers to an anion of formula (alkyl)-SO₃⁻, where alkyl is as defined above. Examples of (C₁-C₆)alkylsulfonate include, but are not limited to, methylsulfonate, ethylsulfonate, propylsulfonate, butylsulfonate, pentylsulfonate, or hexylsulfonate. A preferred (C₁-C₆)alkylsulfonate is methylsulfonate.
As used herein, a "halide" anion refers to a fluoride, a chloride, a bromide or an iodide, preferably a chloride or a bromide, more preferably a bromide.

Unless otherwise indicated, the alkyl, alkenyl, alkynyl, alkoxy, cycloalkyl, heterocycloalkyl, aryl, and heteroaryl groups as defined above can be unsubstituted, or substituted by at least one substituent, for instance by one, two or three substituents. Examples of substituents include, but are not limited to, a (C₁-C₆)alkyl, a (C₂-C₆)alkenyl, a (C₂-C₆)alkynyl, a (C₁-C₆)alkoxy, a (C₃-C₁₂)cycloalkyl, a (C₃-C₁₂)heterocycloalkyl, an aryl, a heteroaryl, a hydroxy, a cyano, a nitro, - SH, or -NH₂.

The present invention relates to a process for extracting double-stranded DNA, comprising the following steps:
(a) reacting a biological sample comprising double-stranded DNA with a compound of formula (I): 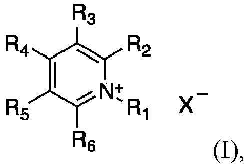 in which:
   - R₁ represents a C₅-C₁₁ alkyl group,
   - R₂, R₃, R₄, R₅ and R₆ represent each independently:
      - a hydrogen atom, a halogen atom, -OH, -CN, -NO₂, -SH, -NR'R" with R' and R" being independently a hydrogen atom or a (C₁-C₆)alkyl, or
      - a radical selected from the group consisting of a (C₁-C₆)alkyl, a (C₂-C₆)alkenyl, a(C₂-C₆)alkynyl, a (C₁-C₆)alkoxy, a (C₃-C₁₂)cycloalkyl, a (C₃-C₁₂)heterocycloalkyl, an aryl, and a heteroaryl, said radical being optionally substituted by at least one -OH, (Ci-C₆)alkoxy, (C₁-C₆)alkanoyl, or -NR'R" with R' and R" being independently a hydrogen atom or a (C₁-C₆)alkyl, and
   - X⁻ represents an anion; and
(b) recovering double-stranded DNA.

In a particular embodiment, R₁ represents a C₇-C₁₀ alkyl group. Preferably, R₁ is an octyl group.

In a particular embodiment, R₂, R₃, R₄, R₅ and R₆ represent each independently a hydrogen atom, or a (C₁-C₆)alkyl optionally substituted by -OH, (C₁-C₆)alkoxy, (C₁-C₆)alkanoyl, or - NR'R" with R' and R" being independently a hydrogen atom or a (C₁-C₆)alkyl.
In a more particular embodiment, R₂, R₃, R₄, R₅ and R₆ represent each independently a hydrogen atom, or a (C₁-C₆)alkyl optionally substituted by -OH, -NH₂, (C₁-C₆)alkoxy, or (Ci-C₆)alkanoyl.
In an even more particular embodiment, R₂, R₃, R₄, R₅ and R₆ represent each independently a hydrogen atom or a (C₁-C₆)alkyl group.

In another particular embodiment, R₂, R₄, and R₆ represent a hydrogen atom.

In a more particular embodiment, R₂, R₄, and R₆ represent a hydrogen atom, and R₃ and R₅ represent each independently a hydrogen atom or a (C₁-C₆)alkyl optionally substituted by -OH, (C₁-C₆)alkoxy, (C₁-C₆)alkanoyl, or -NR'R" with R' and R" being independently a hydrogen atom or a (C₁-C₆)alkyl. In a preferred embodiment, R₂, R₄, and R₆ represent a hydrogen atom, and R₃ and R₅ represent each independently a hydrogen atom or a (C₁-C₆)alkyl.

Preferably, said (C₁-C₆)alkyl is a methyl.

In a particular embodiment, R₃ and R₅ are identical.

Preferably, R' and R" are independently a hydrogen atom or a methyl, more preferably a hydrogen atom.

In a particular embodiment, X⁻ represents an organic or inorganic anion.

Examples of organic anions include, but are not limited to, a carboxylate such as a (Ci-C₆)alkanoate optionally substituted by at least one halogen (preferably fluorine), an oxalate, a citrate, or a tartrate, a sulfonate such as a (C₁-C₆)alkylsulfonate optionally substituted by at least one halogen (preferably fluorine), a benzenesulfonate or toluenesulfonate, an alcoolate such as methanolate or ethanolate, a bis-trifluoromethanesulfonimidate, a dicyanamide (N(CN)₂⁻), a cyanate (OCN⁻), a thiocyanate (SCN⁻), a tris(perfluoroethyl)-trisfluorophosphate, a tetracyanoborate (B(CN)₄⁻), or a tetraphenylborate.

Examples of inorganic anions include, but are not limited to, a halide such as chloride (Cl⁻), bromide (Br⁻), or iodide (I⁻), a perhalate such as a perchlorate (ClO₄⁻), a halate such as a chlorate (ClO₃⁻) or a iodate (IO₃⁻), a nitrate (NO₃⁻), a tetrafluoroborate (BF₄⁻), a tetrachloroaluminate (AlCl₄⁻), a hexafluorophosphate (PF₆⁻), a hexafluoroantimonate (SbF₆⁻).

In a particular embodiment, X⁻ is a halide, a dicyanamide, a nitrate, a cyanate, a thiocyanate, a (C₁-C₆)alkanoate, a (C₁-C₆)alkylsulfonate, a benzenesulfonate or toluenesulfonate.

Preferably X⁻ is a halide, more preferably a bromide or a chloride, even more preferably a bromide.

In a preferred embodiment, a compound of formula (I) is a compound of formula (Ia), or a compound of formula (Ib):

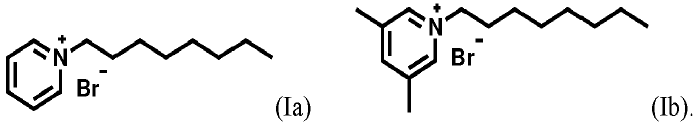

A compound of formula (Ia) can also be referred to as N-octylpyridinium bromide.
A compound of formula (Ib) can also be referred to as 3,5-dimethyl-N-octylpyridinium bromide.

In a more preferred embodiment, a compound of formula (I) is a compound of formula (Ia).

Compounds of formula (I) can be prepared by any method known to the skilled artisan. For instance, such compounds can be prepared by a reaction of a pyridine compound with an alkyl halide, optionally followed by an anion metathesis. This method is in particular described by Papaiconomou et al. (J. Chem. Eng. 2007, 52, 833-840).

### Process

The process of the invention implements the compounds of formula (I) as defined above, for extracting double-stranded DNA. The process comprises:
(a) reacting a biological sample comprising double-stranded DNA with a compound of formula (I) as defined herein; and
(b) recovering double-stranded DNA.

As used herein, the expression "extracting double-stranded DNA" refers to the recovery of all or part of double-stranded DNA contained in a biological sample. Extracting said DNA allows to isolate said DNA from the rest of said biological sample. The expression "purifying double-stranded DNA" may equivalently be used herein for "extracting double-stranded DNA".

As used herein, "double-stranded DNA" refers to a DeoxyriboNucleic Acid molecule having two anti-parallel strands. Said double-stranded DNA may be linear or circular. Circular double-stranded DNA may also be referred to as "plasmid". In a particular embodiment, the double-stranded DNA is a bisulfited DNA.

The process of the invention allows to extract about 10 times more bisulfited double-stranded DNA than a process implemented with commercial kits known in the art, such as Epitect Kit.

In a particular embodiment, the double-stranded DNA has more than 50 base pairs, preferably more than 100 base pairs, more preferably more than 300 base pairs, even more preferably more than 1000 base pairs. Preferably, the double-stranded DNA has less than 30000 base pairs, preferably less than 10000 base pairs, more preferably less than 5000 base pairs.

Step (a) comprises reacting a biological sample comprising double-stranded DNA with a compound of formula (I) as defined herein.

The biological sample of step (a) may be any biological sample comprising double-stranded DNA. In particular, the biological sample may be obtained from a human, animal, vegetal, bacterial, algal, fungal, protozoal, or viral source. The biological sample may be obtained from microbial fermentation, cellular cultures, biological tissues, and/or biological body fluids. Examples biological body fluids are blood, sputum, lymph fluid, cerebrospinal fluid, urine, serum, plasma, sweat, various aspirates. Examples of biological tissues are muscular tissues, bone tissues, mucosa, corneum, skin tissues, epithelium, connective tissues, or neural tissues. Cells of the biological sample may be eukaryotic or prokaryotic. Examples of cells are chondrocytes, osteoblasts, fibroblasts, blood cells, plasmocytes, neurons, hepatocytes, enterocytes, or cancer cells.

In a particular embodiment, the biological sample is selected from a cell lysate, a cell digest, blood, a serum sample and a plasma sample.

The biological sample of step (a) can be prepared by any techniques known to the skilled artisan. It is understood that the process of the invention is carried out under *in vitro* conditions.

The biological sample subjected to step (a) of the process according to the invention, is used under diluted conditions. In a particular embodiment, the double-stranded DNA concentration is comprised between 1 and 20 ng/mL, preferably between 5 and 10 ng/mL.
Step (a) is advantageously carried out at room temperature. As used herein, the expression "room temperature" refers to a temperature comprised between 5 °C and 40 °C, preferably between 15 °C and 30 °C.

The compound of formula (I) may be used pure or in solution. Preferably, the compound of formula (I) is used in solution, more preferably in an aqueous solution.

In a particular embodiment, step (a) comprises the compaction of double-stranded DNA contained in the biological sample.
As used herein, a "compaction" of double-stranded DNA refers to a dehydration of double-stranded DNA. The resulting DNA (i.e. a compacted double-stranded DNA) is insoluble in water. The double-stranded DNA obtained in step (a) is insoluble in water.

Step (a) produces an aggregate (also called herein a "precipitate"). Preferably, this aggregate comprises double-stranded DNA and a compound of formula (I) in the form of a complex. A "complex of double-stranded DNA and a compound of formula (I)" refers to a molecular entity wherein the double-stranded DNA and a compound of formula (I), in particular the pyridinium of said compound, interact with each other, for instance through ionic interactions.

Advantageously, the process, and more particularly step (a), is DNA-selective. The expression "DNA-selective" refers to the ability of a compound of formula (I) to selectively react with double-stranded DNA. In particular, in a double-stranded DNA-selective process, the compound of formula (I) does not substantially react with other biological molecules, such as proteins, RNA, or single-stranded DNA, which may be present in the biological sample. In a preferred embodiment, the compound of formula (I) only reacts with double-stranded DNA.

Step (b) of the process of the invention comprises recovering double-stranded DNA.

In a particular embodiment, step (b) comprises the following steps:
(b1) purifying the aggregate obtained in step (a); and
(b2) contacting the aggregate obtained in step (b1) with a buffer solution.

The expression "purifying an aggregate" refers to the recovery of said aggregate. The expression "isolating an aggregate" can be equivalently used herein.

In a preferred embodiment, the purifying step (b1) comprises a centrifugation step.
Suitable conditions of centrifugation, in particular the temperature, and the centrifugation speed and time, can be determined by the skilled artisan. For instance:
- the temperature may be comprised between 0 °C and 15 °C;
- the centrifugation speed may be comprised between 3000 rpm and 10000 rpm;
- the centrifugation time may be comprised between 1 min and 120 min.
Centrifugation in step (b1) produces said aggregate and a liquid phase (also called "supernatant"). Said supernatant can be removed by any technique known to the skilled artisan, for instance by means of a pipette.
In a particular embodiment, the aggregate isolated in step (b1) is obtained in the form of pellets.

Step (b2) comprises contacting the aggregate obtained in step (b1) with a buffer solution.

The buffer solution used in step (b2) is advantageously alcohol-soluble. In a particular embodiment, said buffer solution contains at least one alkali salt. Examples of alkali salt are a lithium salt, a sodium salt, or a potassium salt, preferably a lithium salt or a sodium salt, more preferably a sodium salt. Examples of sodium salt are sodium chloride or sodium phosphate. Examples of buffer solutions include, but are not limited to, phosphate-buffered saline solution, carbonate buffer solution, citrate buffer solution, monochloracetate buffer solution, acetate, buffer solution, or borate buffer solution. Preferably, the buffer solution is a phosphate-buffered saline solution (also referred to as "PBS" solution).

In a particular embodiment, step (b2) comprises the un-compaction of the double-stranded DNA from the aggregate obtained in step (b1).
As used herein, an "un-compaction" (or "decompaction") of double-stranded DNA refers to a hydration of double-stranded DNA. The resulting DNA (i.e. an un-compacted double-stranded DNA) is soluble in water or in the buffer solution.

In a particular embodiment, the process further comprises:
(b3) contacting the mixture obtained in step (b2) with at least one alcohol solution.

Steps (b2) and (b3) may be carried out simultaneously or successively. Preferably, steps (b2) and (b3) are carried out successively.

In a more particular embodiment, step (b3) comprises the following substeps:
(b3-1) adding a first alcohol solution to the mixture obtained in step (b2);
(b3-2) isolating the aggregate obtained in substep (b3-1); and
(b3-3) optionally, washing the aggregate obtained in substep (b3-2) with a second alcohol solution.

In a particular embodiment, substep (b3-1) comprises the compaction of the double-stranded DNA obtained in step (b2). Substep (b3-1) produces an aggregate, which comprises double-stranded DNA.

Preferably, substep (b3-2) comprises a centrifugation step.

In a particular embodiment, substeps (b3-1) and (b3-2) are cyclically implemented several times, such as two, three or four times, preferably two times. For instance, according to this embodiment, the liquid phase produced by the centrifugation in substep (b3-2) (also referred to as "supernatant") is contacted with said first alcohol solution, and the aggregate obtained therefrom is isolated, preferably by centrifugation.

In a particular embodiment, the alcohol in the alcohol solution used in steps (b3), (b3-1), or (b3-3) is methanol, ethanol, propanol, isopropanol and/or a mixture thereof. Preferably, said alcohol is ethanol.

In a preferred embodiment, the alcohol solution is an aqueous solution. According to this preferred embodiment, the alcohol solution comprises an alcohol as defined herein and water. In a particular embodiment, the alcohol concentration in the alcohol solution is greater than or equal to 50 % (v/v), preferably greater than or equal to 70 % (v/v).

In particular, the alcohol concentration of said first alcohol solution in step (b3-1) may be greater than or equal to 90 % (v/v), and more particularly, greater than or equal to 95 % (v/v). In a preferred embodiment, the first alcohol solution in step (b3-1) is an aqueous solution of ethanol having a concentration greater than or equal to 95 % (v/v).

In particular, the alcohol concentration of said second alcohol solution in step (b3-3) may be comprised between 50 % and 95 % (v/v), and more particularly between 60 % and 80 % (v/v). In a preferred embodiment, the second alcohol solution in step (b3-3) is an aqueous solution of ethanol having a concentration between 60 % and 80 % (v/v).

The optional washing step (b3-3) can be implemented several times, for instance, two times or three times. The washing steps may be separated by centrifugation steps.

In a particular embodiment, the process of the invention comprises the following steps:
(a) reacting a biological sample comprising double-stranded DNA with a compound of formula (I) as defined herein, to form an aggregate;
(b1) purifying the aggregate obtained in step (a); and
(b2) contacting the aggregate obtained in step (b1) with a buffer solution.

In a more particular embodiment, the process of the invention comprises the following steps:
(a) reacting a biological sample comprising double-stranded DNA with a compound of formula (I) as defined herein, to form an aggregate;
(b1) purifying the aggregate obtained in step (a);
(b2) contacting the aggregate obtained in step (b1) with a buffer solution; and
(b3) contacting the mixture obtained in step (b2) with at least one alcohol solution.

In an even more particular embodiment, the process of the invention comprises the following steps:
(a) reacting a biological sample comprising double-stranded DNA with a compound of formula (I) as defined herein, to form an aggregate;
(b1) purifying the aggregate obtained in step (a);
(b2) contacting the aggregate obtained in step (b1) with a buffer solution;
(b3-1) adding a first alcohol solution to the mixture obtained in step (b2);
(b3-2) isolating the aggregate obtained in substep (b3-1); and
(b3-3) optionally, washing the aggregate obtained in substep (b3-2) with a second alcohol solution.

In another particular embodiment, the process of the invention comprises the following steps:
(α) compacting double-stranded DNA from a biological sample in the presence of a compound of formula (I) as defined herein;
(β) un-compacting double-stranded DNA from step (α) in the presence of a buffer solution, preferably a PBS solution;
(γ) re-compacting double-stranded DNA from step (β) in the presence of an alcohol solution, preferably ethanol; and
(δ) recovering double-stranded DNA.

The process of the invention allows to extract double-stranded DNA. The double-stranded DNA extracted by a process according to the invention can be preserved in a dry state or in any suitable solution, such as water, or a buffer (e.g. Tris-EDTA buffer).

The native structure of the double-stranded DNA extracted by a process according to the invention, is substantially preserved. The preservation of the DNA structure can be measured by circular dichroism, or any other techniques known to the skilled artisan.
The extraction yield may be greater than or equal to 40 %, preferably greater than or equal to 60 %, more preferably greater than or equal to 80 %. The extraction yield refers to the ratio of the weight of double-stranded DNA extracted from a biological sample by a process of the invention to the total weight of double-stranded DNA initially contained in said biological sample.

An object of the invention is a method for extracting at least 40 %, preferably 60%, even more preferably 80%, of double-stranded DNA from a biological sample comprising the following steps of:
(a) reacting a biological sample comprising double-stranded DNA with a compound of formula (I), and
(b) recovering double-stranded DNA.

The process of the invention may be implemented under manual or automated conditions. For automated conditions, steps of the process may be carried out by means of well plates comprising membranes. Such membranes can resist organic solvents and allow filtering liquid phases and isolating solid phases. The removal of said liquid phase can be achieved by using a vacuum system (e.g. a pump) connected to the well plates.

Another object of the invention is a use of a compound of formula (I) as defined herein, for extracting double-stranded DNA.

Another object is a kit for extracting double-stranded DNA comprising a composition comprising a compound of formula (I) as defined herein.

In a particular embodiment, the kit further comprises a buffer solution as defined herein, and at least one alcohol solution, as defined herein.

In a preferred embodiment, the kit of the invention comprises:
- a composition comprising a compound of formula (I) as defined herein;
- a buffer solution, preferably a phosphate-buffered saline solution; and
- at least one alcohol solution, preferably comprising ethanol.
More preferably, the kit further comprises an instruction guide.

Each composition or solution of the kit is advantageously contained in an individual compartment.

The kit of the present invention is particularly suitable for implementing a process of the invention.

A further object of the present invention is a use of a kit as defined herein for extracting double-stranded DNA.

The invention will also be described in further details in the following examples, which are not intended to limit the scope of this invention, as defined by the attached claims.

### EXAMPLES

### Methods and Materials

The Phosphate Buffer Saline pH 7.2 contained 150 mM NaCl and 150 mM sodium phosphate. Calf-thymus DNA or "Ct-DNA" had an average size of 1800 bp and a verified high purity: 260nm/280nm= 1.9, and was used diluted in water at 1 µg/ml.

Screened ionic liquid-type compounds described in Example 2 were prepared as previously described in Viboud et al. (J. Hazard. Mater. 2012, 40-48, 215-216).

The 1M aqueous working solution of compound (Ia) was kept at 4°C for 12 months without demonstrating instability.

Confluent MCF7 cells were scrapped in PBS from confluents T25 flasks with a rubber policeman and were next collected by centrifugation. Digests were prepared from MCF7 pellets, mice kidneys and from *vitis vinifera* leaves after digestion. One mg of each tissue was cut in small parts with scissors and immerged in 1 ml of lysis buffer (NaCl 5 M: 20 µl, Tris-HCl pH 8 1M: 10 µl, EDTA (Ethylene Diamine Tetra-Acetic 0.5 M pH 8: 50 µl and water: 910 µl) containing 5 µl Triton X and 5 µl of proteinase K aqueous solution (20 mg/ml). The tubes containing the digests were left in a water bath for 12 h at 55 °C. 900 µL of each digest were further treated by the process of the invention.

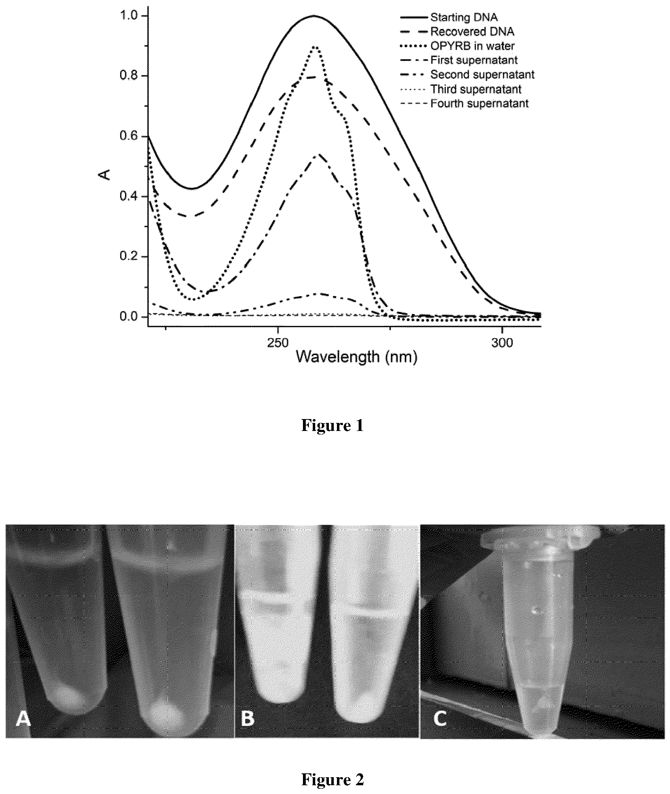

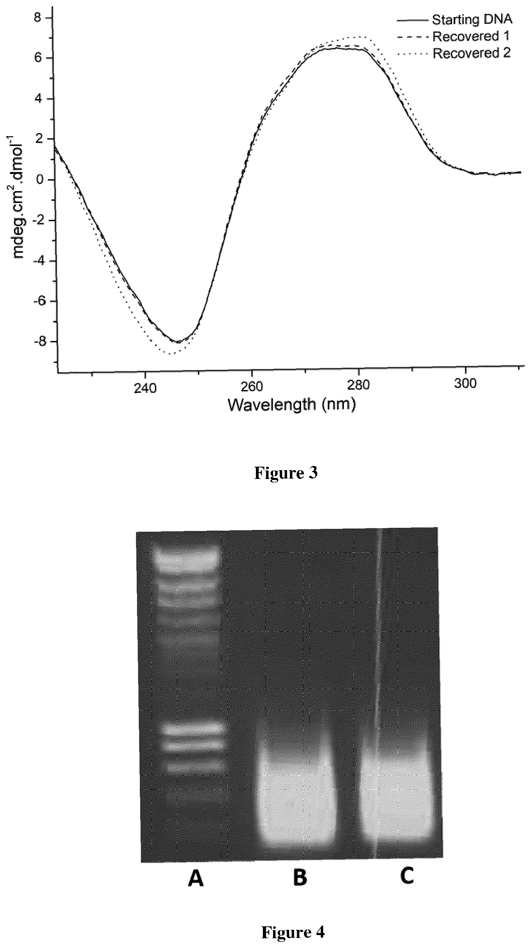

*DNA dosages:* a Cary 100 bio spectrophotometer was used for DNA dosage according to the manufacturer recommendations. The process of the invention was also surveyed by measuring all supernatants absorbance's at 260 nm as shown in Figure 1.
*Circular dichroism:* Circular dichroism experiments were recorded at 20 °C on a Jasco J-810 instrument (Japan). All spectra were run in duplicate with 2 µg of the Ct-DNA obtained by the process of the invention using compound (Ia) and of Ct-DNA diluted in 500 µL PBS (Figure 3).
*Agarose gel electrophoresis analysis:* 1, 2% agarose gel was run at 60 V for 60 min. The running buffer was TAE in which SYBR green was dissolved (TAE 50X at pH 8.5 i.e. Tris-base 0.04 M, acetic acid: 57.1 ml, Ethylene Diamine Tetra-Acetic 0.001 M in water). 3 µl of an aqueous solution of SYBR green (10 mg/ml) is added to 100 ml of tepid agarose in TAE IX. The DNA samples were mixed with 6 µL of sample medium (Sodium Dodecyl Sulfate: 1 % v/v, bromophenol blue: 0.1% v/v, 0.1 M EDTA and 50 % v/v glycerol). The gels are examined under UV light and photographed with a numeric camera for archiving (MW= Molecular weight standards Promega, France).
*DNA quality and qPCR:* the DNAqual and Mitoc kits from Eurobio, France was used to evaluate the processed DNA quality (Bio-Rad qPCR (CFX96) machine). Briefly, the PCR mix contains 2 taqman probes, one being a calibrator (labelled with Texas red) and the other a target gene (labelled with Yakima yellow). For DNAqual the target genomic DNA is Epidermal Growth Factor 1, for Mitoc it is the mitochondrial genome. The experiments are run according to the manufacturer's instructions using the Bio-Rad amplifications reagents and vessels. For *vitis vinifera* a qPCR using SYBR Green was realized, the primers were chosen in the sequence of the ribosomal DNA: 5'-GAGAAACGGCTACCACATCCAAGG-3' (SEQ ID No: 1) and 5'-CCATGCACCACCACCCATAGAATC-3' (SEQ ID No: 2).

### Example 1. Process for extracting double-stranded DNA according to the invention

At room temperature, 100 µl of a 1 M aqueous solution of a ionic liquid-type compound was added to a biological sample (900 µL of all cell digests, bacterial lysates, Ct-DNA and MCF7 DNA bisulfited samples (with the EpiTect Bisulfite Kit and after the conversion step) and also directly to 900 µL of fresh and frozen sera and plasma). Obtained DNA aggregates were pelleted by 10 min centrifugation at 4 °C and 800 x g. Each pellet was then vortexed vigorously within 100 µL of added PBS and next re-condensed with addition, in the same tube, of 600 µL of absolute molecular grade EtOH (EtOH 100%). As before, DNA aggregates were again pelleted by centrifugation. Pellets were finally washed twice with 500 µL of an EtOH/water solution (70 % / 30 %, v/v). The last pellet was left 5 min to dry at room temperature before addition of 100 µL of molecular grade water or pH 8.0 TE buffer.

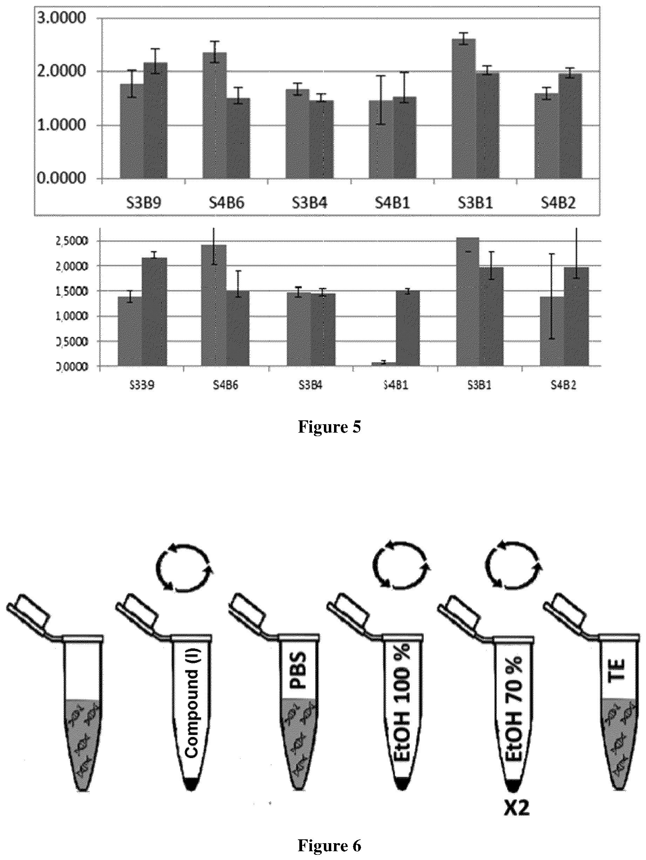

Figure 6 shows a scheme illustrating a process for extracting double-stranded DNA according to the invention (the circular symbol represents a centrifugation step; pH 8.0 TE buffer [10 mM Tris · HCl (pH 8.0), 1 mM EDTA (pH 8.0)]; EtOH = ethanol.

**Table 1**

| Ionic liquid-type compound | % Ct-DNA recovery |
|---|---|
| 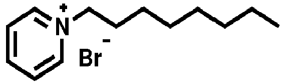 | 80 % |
| 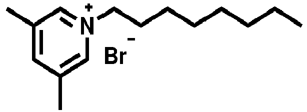 | 40 % |

Figure 1 shows UV spectra of 10 µg Ct-DNA (starting DNA), and 10 µg Ct-DNA after implementing the process as described above (recovered DNA). Absorbance (A) was measured for compound (Ia) 300 µM aqueous solution and for the first, second, third and fourth supernatants obtained during the process. Compound (Ia) absorbance maximum is alike absorbance of DNA. This could be a pitfall since it disturbed DNA dosage, however, it allowed the follow up of the needed efficient Compound (Ia) stripping. This is almost achieved after the second wash as seen in the second supernatant after the second Ct-DNA compaction with absolute EtOH. It is complete after the fourth 70 % EtOH/30 % water (v/v) wash.

A DNA-recovery yield of 80 % was obtained with Compound (Ia), and 40 % with Compound (Ib).
For short DNAs (100-300 bp), the DNA recovery yield reached 60 % with Compound (Ia).

Figure 2 shows some pictures of purified DNA obtained by at different stages of the process as described above, starting from frozen mouse sera and plasma samples without preconditioning, and from plasmid lysates:
- Figure 2A: 900 µL samples of mouse serum or plasma were mixed with 100 µL of an aqueous solution of 1 M Compound (Ia) (after the first centrifugation);
- Figure 2B (right tube): 900 µL of lysed bacterial plasmids mixed with 100 µL 1M Compound (Ia) aqueous solution (after the first centrifugation). Figure 2B demonstrates that the compound used the process of the invention is DNA-selective.
- Figure 2C: MCF7 cells digest after the last centrifugation. Figure 2C demonstrates that all DNA are processable with the process of the invention, from serum, plasma, bacteria and human cells.

### Structure of the DNA extracted by the process of the invention

Figure 3 shows circular dichroism spectra of 2 µg of Ct-DNA (starting DNA) and 2 µg Ct-DNA duplicate obtained by the process of the invention (recovered 1 and 2). Two extremes were observed in spectra: one negative at ∼ 248 nm and the other positive at ∼ 275 nm. Circular dichroism demonstrates that recovered Ct-DNAs were as Ct-DNA in a B-conformation. This indicates that the process of the invention does not change the DNA conformation.

As shown in Figure 4, Ct-DNA and the duplicate obtained by the process of the invention were compared through an agarose gel electrophoresis. This analysis demonstrates an apparent identity between the two samples.

### Example 2. Comparative study

### a) Nature of the ionic liquid-type compound

The process of the invention described in Example 1 was carried out using the following compounds, having various nitrogen-based rings and various alkyl chains:
- pyrrolidinium and piperidinium compounds: 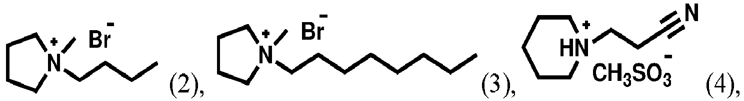 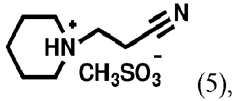
- imidazolium compounds: 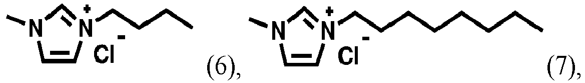
- a pyridinium compound, having a C₄ alkyl chain: 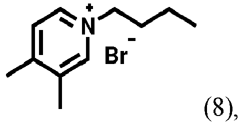
- pyridinium compounds, having C₁₄ and C₁₆ alkyl chains: 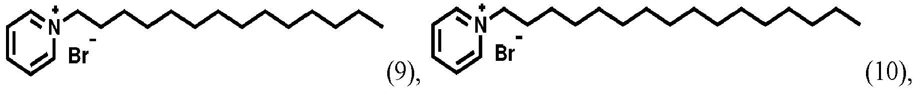
- a dimeric pyridinium compound:

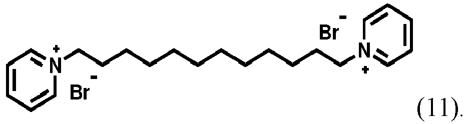

Contrary to the compounds of the invention, none of the above compounds were able to extract double-stranded DNA, selectively and/or with high yields.

Also, Figure 2B (left tube) shows 900 µL of lysed bacterial plasmids mixed with 100 µL 1M Cetyl pyridinium aqueous solution, after the first centrifugation. Contrary to compounds used in the present invention, cetyl pyridinium (C₁₆ alkyl chain) reacts with plasmid proteins as well as plasmid DNA, and is thus not DNA-selective.

### b) Nature of the process

• Table 2 shows DNA UV-absorbance and absorbance ratio (260 nm/280 nm) obtained with the process of the invention as described in Example 1 and known DNA purifications processes. Concentrations variations were of about 5 % with 3 different measurements.
Duplicates samples were processed with the process of the invention of Example 1 (A), with the phenol/chloroform/isoamyl alcohol (25/24/1, v/v) process (B), or with the Qiagen kit (QIAamp DNA Mini Kit, QIAprep Spin Miniprep Kit, DNAeasy Plant Mini Kit) (C). Leaves of *vitis vinifera* were digested in duplicate and impurities were removed before further processing by either method by the first Qiagen column.

**Table 2**

| Process | DNA type | DNA Concentration (ng/(µL) | 260 nm | 280 nm | 260/280 nm |
|---|---|---|---|---|---|
| A | Purified mouse kidney DNA | 840 | 0.458 | 0.365 | 1.40 |
| B | Purified mouse kidney DNA | 740 | 0.338 | 0.331 | 1.02 |
| A | Plasmid purified | 7792 | 1.558 | 0.944 | 1.65 |
| C | Plasmid purified | 3989 | 0.797 | 0.584 | 1.36 |
| A | MCF7 DNA purified | 8 800 | 0.352 | 0.264 | 1.33 |
| C | MCF7 DNA purified | 1230 | 0.246 | 0.211 | 1.16 |
| A | *Vitis vinifera* leaves purified | 1 840 | 0.269 | 0.248 | 1.08 |
| C | *Vitis vinifera* leaves Qiagen purified | 1 350 | 0.217 | 0.204 | 0.83 |

Table 2 shows that the process of the invention (A) is always more efficient that the corresponding Qiagen kit, and also better than the classical phenol/chloroform/isoamyl alcohol process.
• Table 3 shows DNA extraction from mouse and human sera and plasma. Duplicates samples were processed with the process of the invention as described in Example 1 (A) or with the phenol/chloroform/isoamyl alcohol process (B). Concentrations variations were of about 5 % with 3 different measurements.

**Table 3**

| Origins (2 mL samples) | Process B (Total ng) | Process A (Total ng) | Yield (In folds) |
|---|---|---|---|
| Mouse serum 1 | 84 | 1508 | 18 |
| Frozen mouse serum 3 | 37 | 556 | 14.7 |
| Frozen mouse serum 7 | 56 | 818 | 14.4 |
| Mouse plasma 4 | 160 | 1497 | 9.3 |
| Frozen mouse plasma 5 | 42 | 627 | 14.7 |
| Frozen mouse plasma 6 | 77 | 4993 | 64 |
| Human plasma 3 | 173 | 1119 | 6.4 |
| Human plasma 7 | 154 | 711 | 4.6 |
| Human plasma 9 | 115 | 475 | 4.1 |
| Frozen human plasma 6 | 41 | 804 | 19.6 |

The process of the invention (A) is always more efficient than the phenol/chloroform/isoamyl alcohol process (B) implemented here on duplicate samples.
• Figure 5: DNA quality was assessed via qPCR (top of Figure 5: DNA qual - bottom of Figure 5: Mitoc.) In light grey (left part), DNA from mice kidneys obtained by the process of the invention, and in dark grey (right part), phenol/chloroform/isoamyl alcohol purified duplicate DNA. Specific qPCRs were conducted using six different mouse kidney biopsies. Numbers are code for the kidney biopsies.
The quality of Ct-DNAs obtained the process of the invention and by the phenol/chloroform/isoamyl process were equivalent in terms of genomic and mitochondrial DNA.

### c) Nature of the nucleic acid

RNA was prepared from MCF7 cells with the RNAzol kit following the manufacturer's instructions and 100 µg of RNA were subjected to the process as described in Example 1. No pellet appeared.

10 µg of single-stranded DNA were also subjected to the process as described in Example 1, however no pellet appeared.

This demonstrates that the process of the invention using a compound of formula (I) cannot be used for extracting RNA or single-stranded DNA, and is thus selective to double-stranded DNA.

## Claims

1. A process for extracting double-stranded DNA, comprising the following steps:
(a) reacting a biological sample comprising double-stranded DNA with a compound of formula (I): 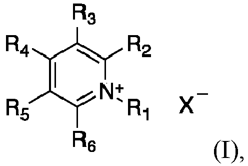 in which:
- R₁ represents a C₅-C₁₁ alkyl group,
- R₂, R₃, R₄, R₅ and R₆ represent each independently:
• a hydrogen atom, a halogen atom, -OH, -CN, -NO₂, -SH, -NR'R" with R' and R" being independently a hydrogen atom or a (C₁-C₆)alkyl, or
• a radical selected from the group consisting of a (C₁-C₆)alkyl, a (C₂-C₆)alkenyl, a (C₂-C₆)alkynyl, a (C₁-C₆)alkoxy, a (C₃-C₁₂)cycloalkyl, a (C₃-C₁₂)heterocycloalkyl, an aryl, a heteroaryl, said radical being optionally substituted by at least one -OH, (Ci-C₆)alkoxy, or (C₁-C₆)alkanoyl, or -NR'R" with R' and R" being independently a hydrogen atom or a (C₁-C₆)alkyl, and
- X⁻ represents an anion; and
(b) recovering double-stranded DNA.

2. The process according to claim 1, wherein R₁ represents a C₇-C₁₀ alkyl group, preferably an octyl group.

3. The process according to claim 1 or 2, wherein R₂, R₃, R₄, R₅ and R₆ represent each independently a hydrogen atom, or a (C₁-C₆)alkyl optionally substituted by -OH, -NH₂, (C₁-C₆)alkoxy, or (C₁-C₆)alkanoyl.

4. The process according to any one of claims 1 to 3, wherein R₂, R₃, R₄, R₅ and R₆ represent each independently a hydrogen atom or a methyl.

5. The process according to any one of claims 1 to 4, wherein X⁻ is a halide, a dicyanamide, a nitrate, a cyanate, a thiocyanate, a (C₁-C₆)alkanoate, a (C₁-C₆)alkylsulfonate, a benzenesulfonate or toluenesulfonate.

6. The process according to any one of claims 1 to 5, wherein X⁻ represents a halide, preferably a bromide or a chloride, more preferably a bromide.

7. The process according to any one of claims 1 to 6, wherein a compound of formula (I) is a compound of formula (Ia), or a compound of formula (Ib):

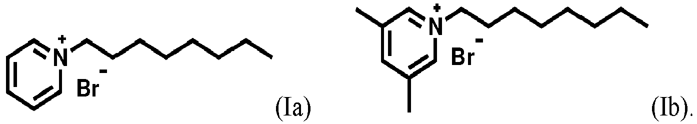

8. The process according to any of claims 1 to 7, wherein the process comprises the following steps:
(a) reacting a biological sample comprising double-stranded DNA with a compound of formula (I) as defined in any one of claims 1 to 7, to form an aggregate;
(b1) purifying the aggregate obtained in step (a); and
(b2) contacting the aggregate obtained in step (b1) with a buffer solution.

9. The process according to claim 8, wherein said buffer solution is a phosphate-buffered saline solution.

10. The process according to claim 8 or 9, wherein the process further comprises:
(b3) contacting the mixture obtained in step (b2) with at least one alcohol solution, preferably comprising ethanol.

11. The process according to claim 10, wherein the alcohol concentration of said alcohol solution is greater than or equal to 70 % (v/v).

12. The process according to any one of claims 11, wherein the double-stranded DNA concentration is between 1 and 20 ng/mL, preferably between 5 and 10 ng/mL.

13. The process according to any one of claims 1 to 12, wherein said double-stranded DNA has more than 50 base pairs, preferably more than 100 base pairs, more preferably more than 300 base pairs, even more preferably more than 1000 base pairs.

14. Use of a compound of formula (I) as defined in any one of claims 1 to 7, for extracting double-stranded DNA.

15. A kit for extracting double-stranded DNA comprising:
- a composition comprising a compound of formula (I) as defined in any one of claims 1 to 7;
- a buffer solution, preferably a phosphate-buffered saline solution;
- at least one alcohol solution, preferably comprising ethanol; and
- optionally an instruction guide.


SEQUENCE LISTING

<110>  CNRS - UCA - Université Savoie Mont Blanc - Université
       Grenoble Alpes - Grenoble INP

<120>  PROCESS FOR EXTRACTING DOUBLE-STRANDED DNA

<130>  B3266EP00

<160>  2

<170>  PatentIn version 3.5

<210>  1
<211>  24
<212>  DNA
<213>  artificial

<220>
<223>  Primer 1

<400>  1
gagaaacggc taccacatcc aagg                                              24


<210>  2
<211>  24
<212>  DNA
<213>  artificial

<220>
<223>  Primer 2

<400>  2
ccatgcacca ccacccatag aatc                                              24